# EUROPEAN PATENT APPLICATION

(11) **EP 1 859 748 A1**
(43) Date of publication of application: **28.11.2007**
(21) Application number: 07252145.3
(22) Date of filing: 24.05.2007
(51) Int. Cl.: A61B 17/15, A61B 17/17, A61B 17/00

(54) **Guide assembly**

(30) Priority: 27.05.2006 GB 0610572
(71) Applicant: Depuy International Limited, Beeston, Leeds LS11 8DT (GB)
(72) Inventor: Colquhoun, Callum, VIC 3160 (AU); Rock, Michael, Leeds-LS13 4NF (GB); Mellor, Grant, Victoria 3806 (AU)
(74) Representative: Belcher, Simon James

(57) **Abstract**

A guide assembly for guiding a bone preparation tool in relation to a patient's bone in an orthopaedic surgical procedure, includes a guide member which has a first face which can be positioned against a surface of the bone which is to be prepared using a bone preparation tool, and an opening extending through the guide member through which the bone preparation tool can be positioned against the bone surface, and which has one of a spigot and a socket formed on it. A location component comprises a locator plate which, when attached to the guide member, extends from the guide member in the direction in which the first face thereof faces, to engage a predetermined feature on the bone so that the guide member can be located relative to the bone surface, and an assembly arm which carries the other of the spigot and the socket so that the guide member and the location component can be connected to one another by engaging the spigot in the socket, after the guide member has been inserted through the surgical incision, so that the location arm extends from the guide member in the general direction in which the first face thereof faces. The locator plate defines a plane where it contacts the bone and the spigot and socket define an assembly axis along which the spigot is moved relative to the socket to be received in the socket. The angle between the locator plate plane and the assembly axis is not more than about 10°.

## Description

This invention relates to a guide assembly, and in particular to a guide assembly for guiding a bone removal tool into a patient's bone in orthopaedic surgery.

In order to prepare a bone for a component of an orthopaedic joint prosthesis, it can be necessary to remove bone tissue. For example, it can be necessary to prepare a hole in the bone to receive a spigot, peg or other projection on the component, or to prepare a planar surface on the bone. A hole and a planar surface can be prepared using a cutting instrument (for example a drill bit, a burr tool or a saw blade) which is inserted through a surgical incision in a patient which provides access to the patient's bone.

The cutting instrument can be guided by a guide assembly to accurately control the location, orientation and dimensions of the cut. The guide assembly can be an "L- shaped" member, in which one limb of the member has an opening formed in it to guide the cutting instrument against the surface of the bone, and the other limb of the member serves as a point of reference to contact the bone so that the guide assembly can be positioned as desired against the bone.

It can be important to minimise the size of the incision, especially in order to reduce the period of time taken for the patent to recover from the surgery.

The present invention provides a guide assembly which comprises a guide member and a location arm which can be releasibly connected to one another once they have been inserted though the surgical incision.

Accordingly, in one aspect, the invention provides a guide assembly for guiding a bone preparation tool in relation to a patient's bone in an orthopaedic surgical procedure, comprising:
a. a guide member which has a first face which can be positioned against a surface of the bone which is to be prepared using a bone preparation tool, and an opening extending through the guide member through which the bone preparation tool can be positioned against the bone surface, and which has one of a spigot and a socket formed on it, and
b. a location component which comprises a locator plate which, when attached to the guide member, extends from the guide member in the direction in which the first face thereof faces, to engage a predetermined feature on the bone so that the guide member can be located relative to the bone surface, and an assembly arm which carries the other of the spigot and the socket so that the guide member and the location component can be connected to one another by engaging the spigot in the socket, after the guide member has been inserted through the surgical incision, so that the location arm extends from the guide member in the general direction in which the first face thereof faces,
in which the locator plate defines a plane where it contacts the bone and the spigot and socket define an assembly axis along which the spigot is moved relative to the socket to be received in the socket, and in which the angle between the locator plate plane and the assembly axis is not more than about 10°.

Providing the guide member and the location arm as separate pieces which can be connected to one another after they have been inserted through the surgical incision can provide significant advantages. The size of the incision required to insert the guide member and location arm separately can be much smaller than the size of the incision required to insert the guide member and location arm as one piece, especially by arranging the angle between the locator plate plane and the assembly axis to be not more than about 10°. This leads to a reduced recovery time, reduced discomfort for the patient, and a reduced risk of infection. As the guide member and location arm can still be connected together after insertion thorough the surgical incision, the location arm can still be used to accurately locate the guide member.

The guide member can have any regular or irregular shape. Frequently, the first face will be approximately planar so that it can be positioned stably against a resected surface of a bone. Preferably, the opening is provided in the planar surface.

The location arm can have any regular or irregular shape. Preferably, the arm is approximately straight when viewed along its length, at least on the surface which faces towards the surface of the patient's bone. Preferably, the first face is substantially planar so that it can be positioned flush against a resected surface of a bone.

Preferably, when assembled, the angle between the surface of the location arm for contacting the bone and the first face of the guide member is at least 45°, more preferably at least 75°, especially preferably at least 85°, for example is 90°. Preferably, when assembled, the angle between the surface of the location arm for contacting the bone and the first face of the guide member is not more than 135°, more preferably not more than 105°, especially preferably not more than 95°.

Preferably, the ratio of the length of the location arm in a direction perpendicular to the first face of the guide member to the length of the guide member in a direction parallel to the first face of the guide member is at least 0.25, more preferably at least 0.5, especially preferably at least 0.75. Preferably the ratio of the length of the location arm in a direction perpendicular to the first face of the guide member to the length of the guide member in a direction parallel to the first face of the guide member is at most 1.5, more preferably at most 1.0.

The cooperating formations can be any formations which enable the guide member and location arm to be connected to one another and subsequently disconnected. Preferably, one of the location arm and the guide member has a spigot and the other of the location arm and the guide member has a socket in which the spigot can be received. In this case, cooperating formations are provided by the spigot and the socket. Such cooperating formations are particularly advantageous as the guide member and the location arm can easily be connected and disconnected by sliding one into or out of the other.

Preferably, the spigot is provided on the location arm. Preferably, the socket is provided by guide member. Preferably the socket is provided by the opening in the guide member which is provided to guide the bone preparation tool. This reduces the number of openings required to be formed in the guide member and hence reduces the complexity of the guide member. This can in turn reduce the size of the guide member.

Preferably, the cooperating formations restrict relative rotation between the guide member and the location arm. Preferably, the spigot and socket are non-circular. Optionally, there can be at least two spigots and at least two corresponding sockets. Preferably, the at least two spigots are provided by the location arm. In this case, preferably each of the at least two corresponding sockets are openings extending through the guide member through which the bone preparation tool can be positioned against the bone surface.

Preferably, the guide member has a fixation device opening for receiving a fixation device which can extend through the opening into the bone to fasten the guide member to the bone. Preferably, the guide member has a second fixation device opening for receiving a second fixation device which can extend through the opening into the bone to fasten the guide member to the bone. This can be advantageous because it allows the position of the guide member to be fixed relative to the bone by passing a fixation device through the second opening into the bone. Preferably the fixation device openings are suitable for receiving bone pins, to fasten the guide member to the bone.

Preferably, the assembly includes a guide member handle for manipulating the guide member from outside the surgical incision. This is advantageous because it allows for the easy insertion and manipulation of the guide member *in situ,* without the need for the surgeon to insert his fingers through the surgical incision. Preferably, the guide member handle is removably mounted on the guide member. This is particularly advantageous when the assembly includes a location arm handle because once the location arm and guide member have been connected to one another, the guide member handle can be removed and the guide member can still be manipulated by the location arm handle.

Preferably the assembly includes a location arm handle for manipulating the location arm from outside the surgical incision. This is advantageous because it allows for the easy insertion and manipulation of the location arm in situ, without the need for the surgeon to insert his fingers through the surgical incision. Optionally, the location arm and the location arm handle can have cooperating formations which enable them to be releasibly connected to one another.

The opening in the guide member can be a drill guide bore which defines the axis along which a drill bit can cut bone tissue. The opening in the guide member can be a slot which defines a plane on which bone tissue can be cut by a bone saw or burr tool. Preferably, the slot extends along at least 50% of the length of the guide member, in a direction parallel to the first face of the guide member, more preferably along at least 75%, especially preferably along at least 90%.

The opening can be shaped so as to define an axis along which a drill bit can cut bone tissue and so as to define a plane on which bone tissue can be cut by a bone saw or burr tool. Preferably, the part of the opening which defines the axis along which a drill bit can cut bone tissue provides the socket in which the spigot can be received.

The guide member can have at least two openings extending between the first and second sides which define bone tissue which can be removed by a bone removal tool extending through the openings. Preferably, at least one of the openings is a drill guide bore which defines the axis along which a drill bit can cut bone tissue. Preferably, at least one of the openings is a slot which defines a plane on which bone tissue can be cut by a bone saw or burr tool.

Preferably, the guide member and the location arm between them provide a locking mechanism which can be actuated between an unlocked position in which the guide member and the location arm are free to move relative to each other and a locked position
in which the guide member and the location arm when assembled are locked against movement relative to each other. This is advantageous because when the guide member and location arm are locked together they can be moved securely in all directions as a single unit. Preferably, the locking mechanism comprises a locking arm rotatably mounted on one of the guide member and the location arm. Preferably, the locking arm is rotatably mounted on one of the guide member and the location arm at its head end. Preferably the head end of the locking arm has formations which can engage corresponding formations of a tool to enable the locking arm to be rotated between its locked and unlocked positions.
Preferably, the head end of the locking arm has a non-circular socket which can receive a correspondingly shaped tool.

In a second aspect, the present invention provides a kit of parts for use in preparing a bone to receive a component of an orthopaedic joint prosthesis comprising:
a. a guide assembly as discussed above; and
b. a cutting guide having:
   a peg which can fit snugly into an opening in the bone formed using the guide member so as to mount cutting guide on the bone; and
   a surface which defines the location and orientation of the bone to be cut by a cutting tool extending along the surface towards the bone tissue.

It is an advantage of the present invention that the guide assembly can be used to guide a bone removal tool to remove some bone tissue and that the cutting guide can be used to guide a bone removal tool to remove further bone tissue. Using the kit of parts of the invention it is not necessary to provide one guide which has all of the necessary openings and surfaces to guide a bone removal tool to prepare the bone. Accordingly, the sizes of the guide assembly and the cutting guide can each be smaller than the size of a single guide which provides all of the required openings.

It is a further advantage that the cutting guide has at least one peg which can fit snugly into an opening in the bone formed the guide member, so as to mount the cutting guide on the bone. The peg can determine the location of the cutting guide relative to the bone and so in order to properly locate the cutting guide relative to the bone, the surgeon can simply plug the cutting guide into the patient's bone.

Preferably, the cutting guide comprises an opening, the internal wall of which provides the surface. Accordingly, the opening defines the location and orientation of the bone to be cut by a cutting tool extending through the opening towards the bone tissue.

The opening in the cutting guide can be a drill guide bore which defines the axis along which a drill bit can cut bone tissue. The opening in the cutting guide can be a slot which defines a plane on which bone tissue can be cut by a bone saw or burr tool.

Preferably, the cutting guide has at least two openings which define bone tissue which can be removed by a bone removal tool extending through the opening towards the bone tissue.

Preferably the cutting guide has at least two pegs which can fit snugly into respective openings in the bone formed using the guide member, so as to mount the cutting guide on the bone. This is advantageous because this can help determine the rotational orientation of the cutting guide relative to the bone. Preferably at least one of the pegs can fit snugly into a hole in the bone formed by a fixation device extending through the fixation device opening.

The assembly will generally be made from metallic based materials which are conventionally used in the manufacture of surgical instruments. Certain stainless steels can be particularly preferred. However, it will be understood that at least one part of the assembly, for instance the location arm, can be made from polymeric materials. Using polymeric materials can reduce the cost of manufacture of the assembly, especially because an assembly made from polymeric materials can easily be manufactured using a moulding process. Suitable polymeric materials include certain polycarbonates, polyester, polyamides, poly-ether-ether ketones (PEEKs), and polyaryl-ether ketones (PAEKs). Polymeric materials can be reinforced with particulate material, especially fibrous materials, to provide appropriate wear characteristics.

Embodiments of the invention will now be described, by way of example only, and with reference to the accompanying drawings, in which:
Figure 1 shows a perspective view of a guide assembly according to the present invention.
Figure 2 shows a perspective view of the guide member of the guide assembly shown in Figure 1.
Figure 3 shows a perspective view of the locating arm of the guide assembly shown in Figure 1.
Figure 4a shows a rear perspective view of a cutting guide according the present invention.
Figure 4b shows a front perspective view of the cutting guide shown in Figure 4a.
Figure 5 shows a side view of the guide member shown in Figure 2 with a guide member handle.
Figure 6 shows a perspective view of the guide member and guide member handle shown in Figure 5.
Figure 7 shows a perspective view of the guide member, guide member handle, and locating arm shown in Figures 1, 2 and 3, before the guide member and locating arm are connected to one another.
Figure 8 shows a perspective view of the cutting guide shown in Figure 4 with an insertion handle.
Figure 9 shows a perspective view of an alternative embodiment of a guide assembly according to the present invention.
Figures 10a and 10b show perspective views of a further alternative embodiment of a guide assembly according to the present invent
Figures 11a and b show perspective views of an alternative embodiment of a cutting guide according to the present invention.

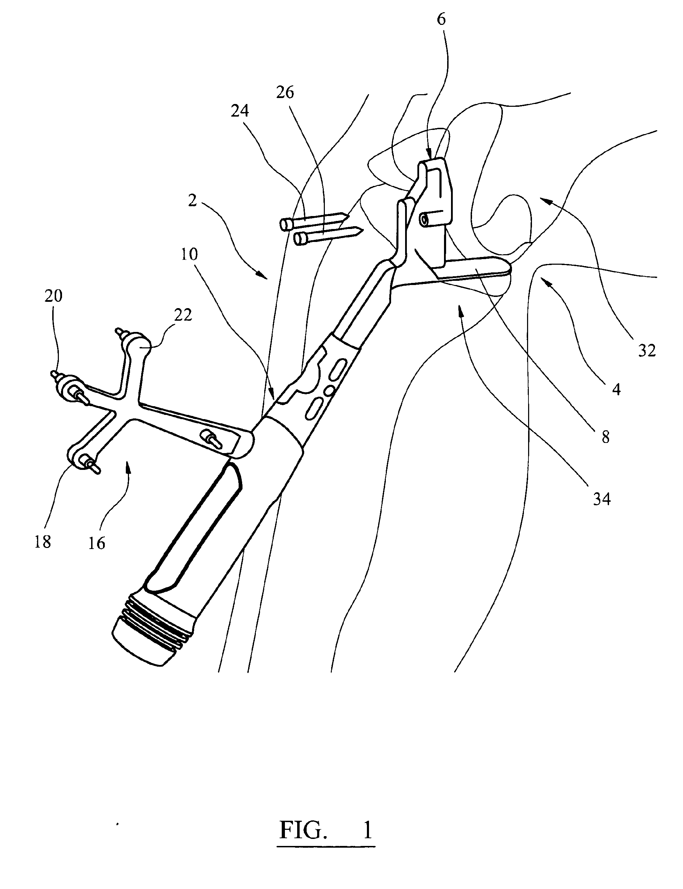

Referring to the drawings, Figure 1 shows a guide assembly 2 according to the present invention inserted through a surgical incision (not shown) at a patient's knee joint 4, between the distal end of the femur 32 and the proximal end of the tibia 34.

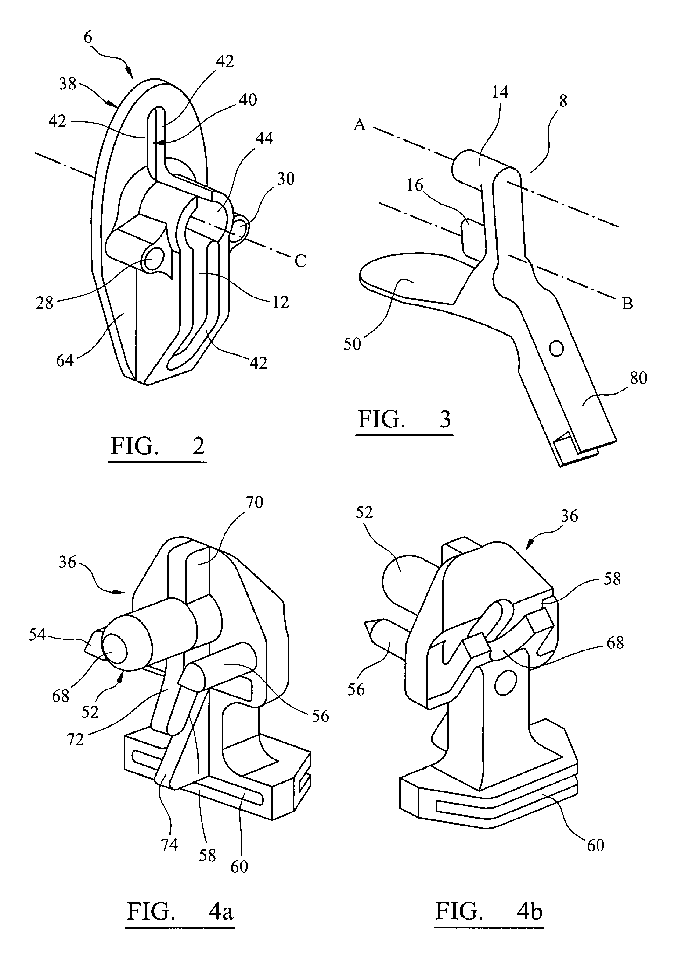

In the embodiments described herein, the guide assembly 2 is used to form a peg hole and a keel slot in a patient's medial femoral condyle to receive a peg and keel of a femoral component of a joint orthopaedic prosthesis. Further, a cutting guide 36 (shown in Figure 4) is used to prepare the posterior cut, posterior chamber cut and the anterior chamber cut on the femur. The guide assembly 2 is to be used subsequent to a distal femoral cut having previously being performed. Nevertheless, it will be understood that the invention is not to be limited such guide assemblies, but can also cover other guide assemblies used to prepare other types of bone which can provide different types of cuts, and need not necessarily require cuts to be made before its use. For instance, the guide assembly 2 and cutting guide 36 can be used to prepare either the medial or lateral condyle. Furthermore, although the guide assembly 2 and cutting guide 36 described are for uni-condylar surgery, it will be understood by the skilled person that the present invention can also be used in guide assemblies for total knee replacement, i.e. multi-condylar surgery.

The guide assembly 2 comprises a guide member 6 and a location arm 8. In the embodiment shown, the location arm 8 has mounting formations 80 for receiving a location arm handle 10. When the location arm handle 10 is connected to the location arm's 8 mounting formations 80 the location arm 8 can be manipulated from outside of the surgical incision. As shown in Figures 1, 2 and 3 the guide member 6 and the location arm 8 are connected to one another by respective cooperating formations 12, 14, 16. Accordingly, when assembled, the guide member 6 and location arm 8 can both be manipulated by the location arm handle 10.

The guide assembly 2 further comprises first 24 and second 26 bone pins which can extend through first 28 and second 30 bone pin openings in the guide member 6, and into the distal end of the femur 32 to fix the guide member 6 to the femur 32.

A reference frame 16 having three arms 18, 20, 22 is attached to the location arm handle 10. The end of each arm 18, 20, 22 has mountings for receiving reference members (not shown). The reference members can be any members that can be tracked by an Image Guided Surgery (IGS). Suitable reference members, their materials, and methods for detecting and calculating their location, for use in IGS systems are well known to a person of ordinary skill in the art and therefore a detailed discussion of such systems is not within this description so as not to obscure the present invention. The use of the reference members and an IGS system can aid the surgeon to determine the location of the guide assembly relative to the patient's body.

Referring to Figure 2, the guide member 6 comprises a first, substantially planar, face 38 which can be positioned against the pre-resected surface of the distal end of the femur 32. The guide member 6 has a slot 40 extending through it. The slot 40 is shaped and sized so that a saw or a burr/mill tool can extend through it to cut an elongate hole in the distal end of the femur 32 against which the first face 38 is positioned. The wall 42 of the slot 40 at its middle is arcuate on both of its sides to define a drill guide 44 through which a drill bit can extend to drill a hole in the distal end of the femur 32 against which the first face 38 is positioned. The drill guide 44 defines a drill guide axis C. The width of the wall 42 of the slot 40 is wider at the drill guide part 44 and along its length towards one of the slot's ends, than the width of the wall 42 from the drill guide part toward the other of the slot's ends. The guide member's 6 cooperating formation is provided by the wider parts 12 of the slot wall 42. The guide member 6 has first 28 and second 30 bone pin openings located on either side of the slot 40, for receiving first 24 and second 26 bone pins.

Referring to Figure 3, the location arm 8 comprises a first, substantially planar, plate 50, and first 14 and second 16 spigots. The first spigot 14 is generally cylindrical and is a snug fit within the drill guide 44 of the guide member 6. The second spigot 16 has opposing first and second planar sides which fit snugly between the wider parts 12 of the wall 42 of the slot 40 of the guide member 6. The location arm 8 further comprises a location arm handle 10, only a portion of which is shown in Figure 3.

The guide member 6 and location arm 8 are configured so that the first 14 and second 16 spigots can be received in the guide member's 6 cooperating formation 12 by moving them in a direction indicated by axes A and B which are parallel to the plane of the first plate 50.

The guide member 6 and location arm 8 are configured such that when assembled, the distance between plane of the first plate 50 and the drill guide axis C is the same as the distance between (a) the plane of a condyle abutment surface of a condyle implant (not shown) wherein the condyle abutment surface is to abut against the surface which the first plate 50 abuts, and (b) an axis of a peg of the condyle implant wherein the peg is to fit in the hole formed by a drill bit extending through the drill guide 44 in the guide member 6.

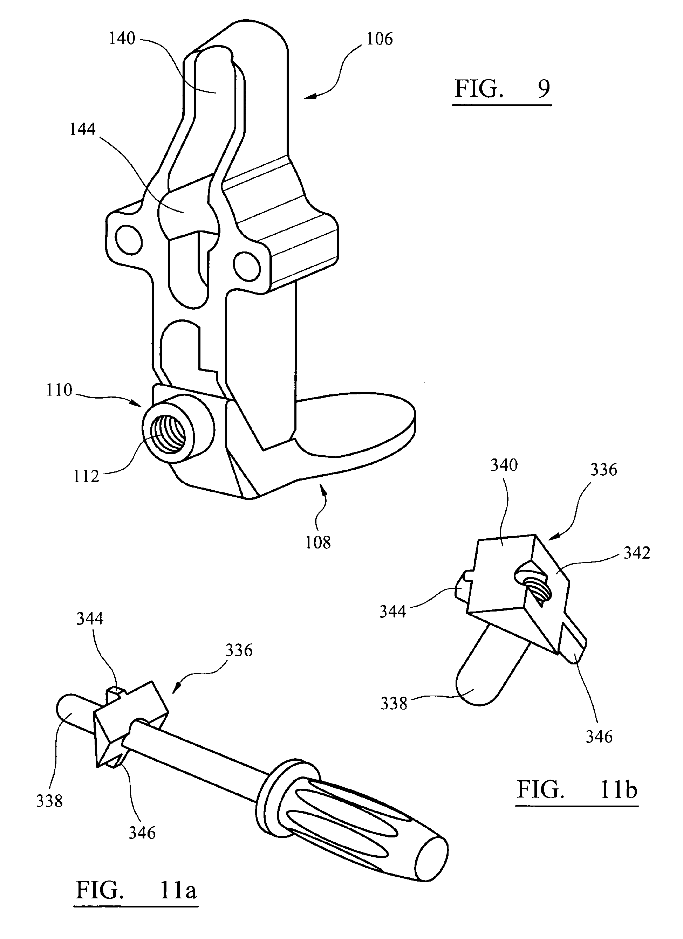

Figure 9 shows an alternative embodiment of a guide member 106 and location arm 108. In this embodiment, the cooperating formations (not shown) are provided by a spigot provided on the location arm 108 and a corresponding socket (not shown) in the guide member 106, which is different to the slot 140 and drill guide 144 in the guide member 106. Further the location arm 108 does not include a location arm handle. However, location arm 8 does provide a mounting in the form of a bore 110 with an internal screw thread 112 by which a location arm handle (not shown) can be releasibly mounted onto the location arm 108.

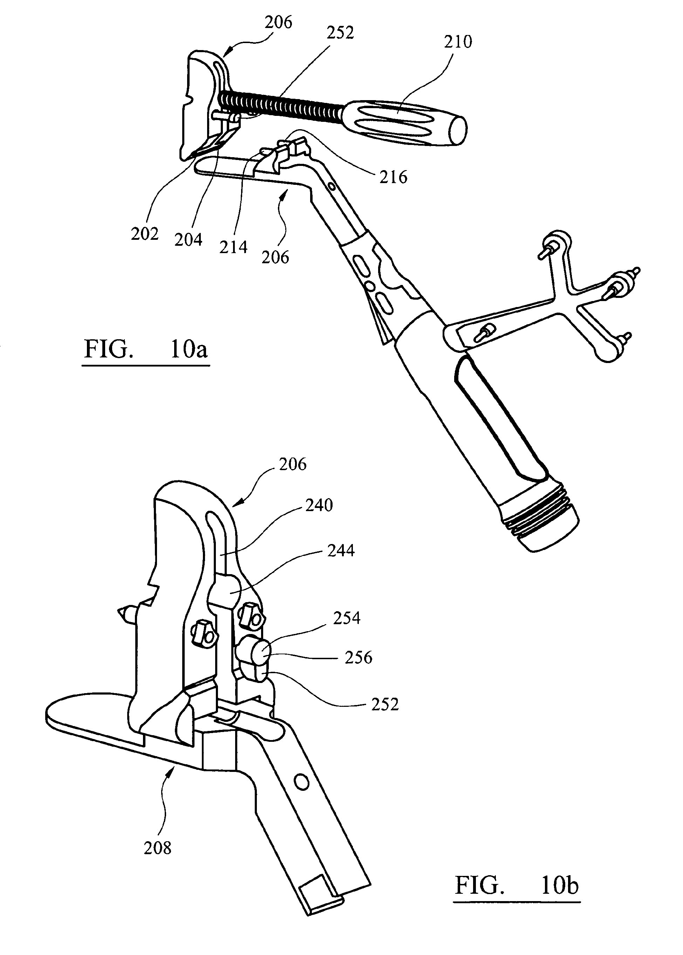

Figures 10a and b show a further alternative embodiment of a guide member 206 and location arm 208. In this embodiment, the cooperating formations are provided by first 214 and second 216 spigots on the location arm 208 and corresponding first 202 and second 204 sockets, both of which are different to the slot 240 and drill guide 244 in the guide member 206. A guide member handle 210 has a spigot (not shown) which can be received in the drill guide part 244 of the slot 240 to enable the guide member handle 210 to be releasibly fastened to the guide member 206.

The guide member 206 has a locking mechanism in the form of a locking arm 252. As shown in Figure 10(b) the locking arm 252 is in its locked position. The locking arm 252 is rotatably mounted on the guide member 206 at the locking arm's 252 head end 254. The head end 254 has a hexagonally shaped socket 254 for receiving a hexagonally shaped tool (not shown) by which the locking arm 252 can be rotated. The length and position of the locking arm 252 is such that when the locking arm 252 is rotated into its locked position as shown in Figure 10b, the locking arm 252 extends over a part of the location arm 208 so that the location arm 208 is unable to slide out from the guide member 206. The locking arm 252 can be rotated to its unlocked position in which the locking arm 252 no longer extends over the location arm 208. When the locking arm 252 is in its unlocked position, the location arm 208 can be slidingly removed from the guide member 206.

Referring to Figures 4a and 4b there is shown a cutting guide 36 according to the present invention. The cutting guide 36 has first 52, second 54 and third 56 pegs, and first 58 and second 60 slots. The first peg 52 can be received in a hole in the distal end of the femur 32 formed by a drill bit extending through the drill guide 44 in the guide member 6. A bore 68 extends through the first peg 52. The second 54 and third 56 pegs can be received in holes in the distal end of the femur 32 formed by the first 24 and second 26 bone pins which extend through the first 28 and second 30 bone pin openings in the guide member 6.

The cutting guide 36 further comprises first 70, second 72 and third 74 projections which can be received in the elongate hole in the bone created by a bone removal tool extending through the slot 40 of the guide member 6. The provision of first 52, second 54 and third 56 pegs and first 70, second 72 and third 74 projections which can be received in holes prepared using the guide member 6 enables the position of the cutting guide 36 relative to the bone to be controlled. Accordingly, it is not necessary to provide additional bone reference features on the cutting guide 36, which would increase the size of the cutting guide 36, in order to allow the cutting guide 36 to be accurately located.

Referring to Figures 11a and 11b there is shown an alternative embodiment of a cutting guide 336 according to the present invention. The cutting guide 336 comprises a peg 338 which can be received in a hole in the distal end of the femur 32 formed by a drill bit extending through the drill guide 44 in the guide assembly. The cutting guide 336 further comprises first 340 and second 342 surfaces each of which can be used to guide a cutting tool to make the chamfer cuts to the distal end of the femur.

The cutting guide 336 also comprises first 344 and second 346 projections which can be received in the elongate hole in the bone created by a bone removal tool extending through the slot 40 of the guide member 6. The first 344 and second 346 projections enables the position of the cutting guide 336 relative to the bone to be controlled. Accordingly, it is not necessary to provide additional bone reference features on the cutting guide 336, which would increase the size of the cutting guide 336, in order to allow the cutting guide 336 to be accurately located.

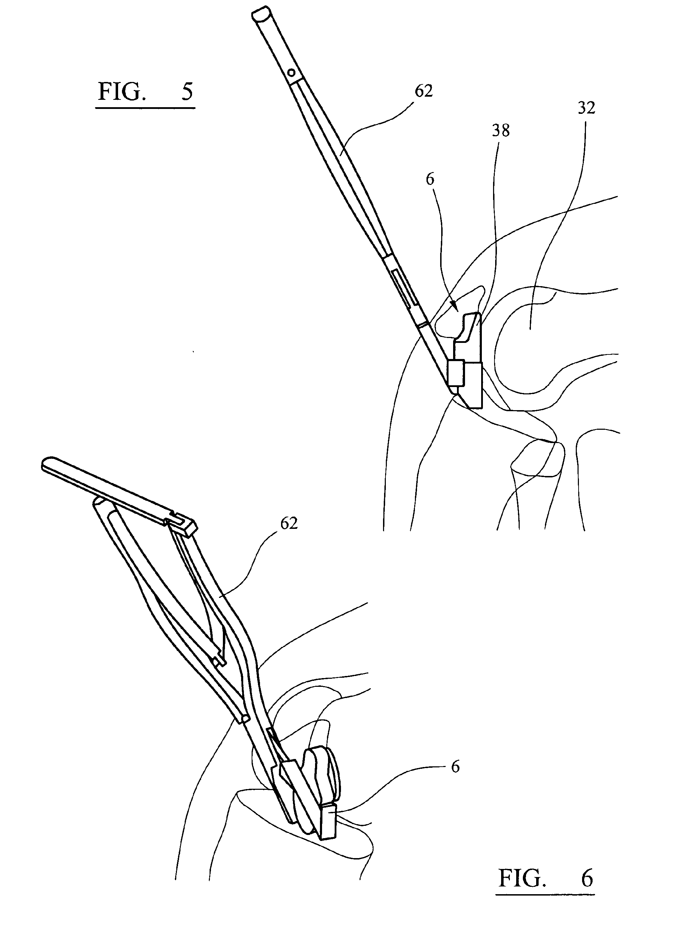

In use, as shown in Figure 5, the guide member 6 is inserted through the surgical incision (not shown) until the first face 38 of the guide member 6 rests flush against a pre-resected surface of the distal end of the femur 32. The guide member 6 can either be manipulated by the surgeon by the surgeon holding the guide member 6 directly, or by connecting a guide member handle 62 to the guide member 6 and then manipulating the guide member 6 via the guide member handle 62. In the embodiment shown in Figures 5 and 6, the guide member handle 62 is fastened to the guide member 6 by sliding first and second recess (not shown) at a first end of the guide member handle 62 over first 64 and second (not shown) flanges on the guide member 6. However, as will be understood, the guide member handle 62 can be releasibly connected to the guide member 6 by any suitable cooperating formations. Indeed, as shown in Figure 10a, a guide member handle 210 can be fastened to a guide member 206 via a spigot and socket configuration.

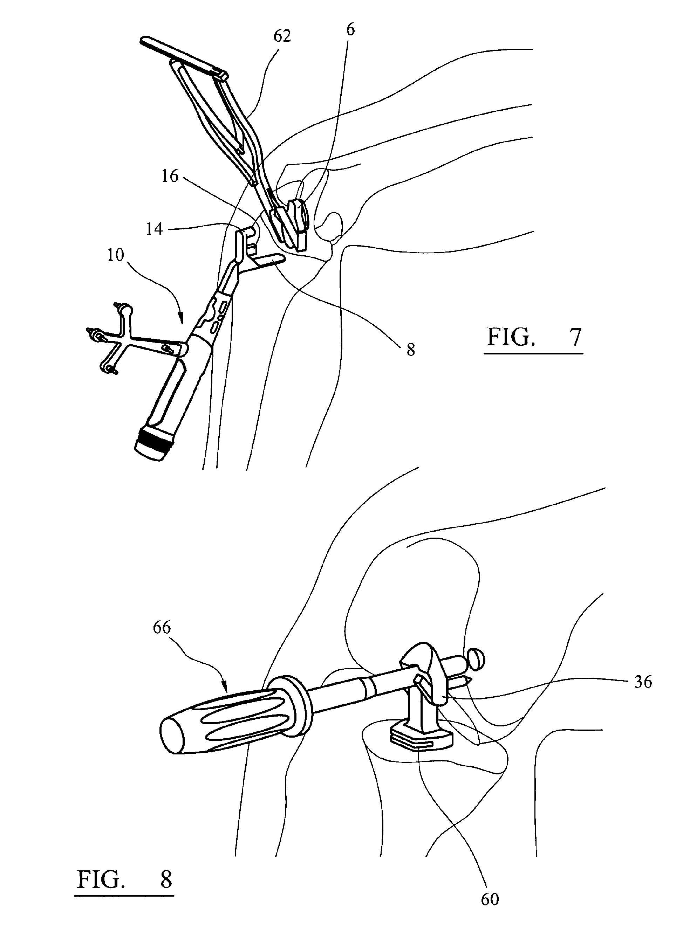

Once the guide member 6 has been inserted through the surgical incision, the location arm 8 is then inserted through the surgical incision as shown in Figure 7. The location arm 8 is then connected to the guide member 6 by locating the first 14 and second 16 spigots in the guide member's 6 cooperating formation 12, which is provided by the wider parts of the slot 40. Once connected, both the guide member 6 and the location arm 8 can be manipulated together using the location arm handle 10. Accordingly, the guide member arm 62 can then be disconnected from the guide member 6, as shown in Figure 1. Due to the first spigot axes A and B and the drill guide axis C being parallel to the plane of the first plate 50, the location arm 8 can be inserted through the surgical incision, and connected to the guide member 6, by moving the location arm 8 in one direction only. That is in a direction parallel to the first 14 and second 16 spigot axes A and B, drill guide axis C and the plane of the first plate 50.

The guide member 6 is then moved via the location arm handle 10 until the guide member 6 is in its desired position. To aid positioning of the guide member 6 relative to the distal end of the femur 32, the location arm 8 is engaged against the medial condyle of the femur.

Once the guide member 6 is in the desired position, the guide member 6 can be fixed to the distal end of the femur 32 by inserting first 24 and second 26 bone pins through the first 28 and second 30 bone pin openings. The location arm 8 can then be disconnected from the guide member 6 by pulling the first 14 and second 16 spigots away from the guide member 6, and the location arm 8 can be subsequently withdrawn through the surgical incision.

Bone removal tools (not shown) can then be inserted through the slot 44 and the drill guide 44 to remove bone from the distal end of the femur 32. In particular, a saw or burr/mill tool can be inserted through the slot 40 to cut an elongate hole in the femur, and a drill bit can be inserted through the drill guide 44 to cut a bore in the femur.

After the elongate slot and the bore have been formed in the femur, the guide member 6 can be detached from the bone and withdrawn through the surgical incision. The cutting guide 36 can then be inserted through the surgical incision and manipulated via a cutting guide handle 66, as shown in Figure 8. The cutting guide handle 66 has a spigot (not shown) at a first end which can be received in the bore 68 which extends through the first peg 52, so as to allow the cutting guide handle 66 to be releasibly connected to the cutting guide 36. The cutting guide 36 is fastened to the bone by inserting the first peg 52 into the bore formed by the drill bit extending through the drill guide 44 in the guide member 6, and by inserting the second 54 and third 56 pegs into the holes formed by the bone pins 24 and 26. Once inserted, the cutting guide handle 66 can be disconnected from the cutting guide. A saw or burr/mill tool (not shown) can then extend through the first 58 and second 60 slots to resect the distal end of the femur 32. After resection of the femur 32, the cutting guide 36 can be detached from the femur 32 and withdrawn through the surgical incision.

## Claims

1. A guide assembly for guiding a bone preparation tool in relation to a patient's bone in an orthopaedic surgical procedure, comprising:
a. a guide member which has a first face which can be positioned against a surface of the bone which is to be prepared using a bone preparation tool, and an opening extending through the guide member through which the bone preparation tool can be positioned against the bone surface, and which has one of a spigot and a socket formed on it, and
b. a location component which comprises a locator plate which, when attached to the guide member, extends from the guide member in the direction in which the first face thereof faces, to engage a predetermined feature on the bone so that the guide member can be located relative to the bone surface, and an assembly arm which carries the other of the spigot and the socket so that the guide member and the location component can be connected to one another by engaging the spigot in the socket, after the guide member has been inserted through the surgical incision, so that the location arm extends from the guide member in the general direction in which the first face thereof faces,
in which the locator plate defines a plane where it contacts the bone and the spigot and socket define an assembly axis along which the spigot is moved relative to the socket to be received in the socket, and in which the angle between the locator plate plane and the assembly axis is not more than about 10°.

2. A guide assembly as claimed in claim 1, in which the spigot is provided on the location component, and the socket is provided by the opening in the guide member which is provided to guide the bone preparation tool.

3. A guide assembly as claimed in claim 1, in which the spigot and socket are non-circular.

4. A guide assembly as claimed in claim 1, in which the guide member has a second opening for receiving a fixation device which can extend through the opening into the bone to fasten the guide member to the bone.

5. A guide assembly as claimed in claim 1, in which the assembly includes a guide member handle which can be removably mounted on the guide member, for manipulating the guide member from outside the surgical incision.

6. A guide assembly as claimed in claim 1, which includes a location component handle for manipulating the location component from outside the surgical incision.

7. A guide assembly as claimed in claim 1, in which the opening in the guide member is a drill guide bore which defines the axis along which a drill bit can cut bone tissue.

8. A guide assembly as claimed in claim 1, in which the opening in the guide member is a slot which defines a plane on which bone tissue can be cut by a bone saw.

9. A guide assembly as claimed in claim 1, in which the guide member has at least two openings extending between the first and second sides which define bone tissue which can be removed by a bone removal tool extending through the openings.

10. A guide assembly as claimed in claim 1, in which the guide member and the location component between them provide a locking mechanism which can be actuated between an unlocked position in which the guide member and the location component are free to move relative to each other and a locked position in which the guide member and the location component are locked against movement relative to each other.

11. A kit of parts for use in preparing a bone to receive a component of an orthopaedic joint prosthesis comprising:
a. a guide assembly as claimed in claim 1; and
b. a cutting guide having:
a peg which can fit snugly into an opening in the bone formed using the guide member so as to mount cutting guide on the bone; and
a surface which defines the location and orientation of the bone to be cut by a cutting tool extending along the surface towards the bone tissue.

12. A kit of parts as claimed in claim 13, in which the cutting guide comprises an opening which provides the surface and which defines the location and orientation of the bone to be cut by a cutting tool extending through the opening towards the bone tissue.

13. A kit of parts as claimed in claim 14, in which the opening is a slot which defines bone tissue which can be cut by a bone saw.

14. A kit of parts as claimed in claim 14, in which the cutting guide has at least two openings which define bone tissue which can be removed by a bone removal tool extending through the opening towards the bone tissue.
